# EUROPEAN PATENT SPECIFICATION

(11) **EP 3 787 497 B1**
(45) Date of publication and mention of the grant of the patent: **08.01.2025**
(21) Application number: 19797039.5
(22) Date of filing: 06.05.2019
(51) Int. Cl.: A61B 5/06, A61B 5/00, A61B 5/05, A61J 15/00, A61M 25/01

(54) **SYSTEMS AND APPARATUS FOR DELIVERING A MEDICAL DEVICE BEYOND A BIFURCATION IN A BODY LUMEN**
SYSTEME UND VORRICHTUNG ZUR ABGABE EINER MEDIZINISCHEN VORRICHTUNG ÜBER EINE VERZWEIGUNG IN EINEM KÖRPERLUMEN
SYSTÈMES ET APPAREIL DE POSE D'UN DISPOSITIF MÉDICAL AU-DELÀ D'UNE BIFURCATION DANS UNE LUMIÈRE CORPORELLE

(30) Priority: 04.05.2018 US 201862667132 P
(43) Date of publication of application: 10.03.2021
(73) Proprietor: CoapTech, Inc., Baltimore, MD 21230 (US)
(72) Inventor: TROPELLO, Steven P., Baltimore, Maryland 21212 (US); GOLDWASSER, Elisabeth, Baltimore, Maryland 21224 (US); CAROLAN, Howard, Baltimore, Maryland 21224 (US)
(74) Representative: Cooley (UK) LLP
(86) International application number: PCT/US2019/030902
(87) International publication number: WO 2019/213657

(56) References cited:
- US-A- 3 961 632
- US-A- 5 431 640
- US-A1- 2003 040 671
- US-A1- 2009 012 517
- US-A1- 2010 094 116
- US-A1- 2016 081 652
- US-B1- 6 651 665
- US-B2- 6 701 918
- US-B2- 8 226 637

## Description

### Cross-Reference to Related Applications

This application claims priority to and the benefit of U.S. Provisional Application No. 62/667,132, filed May 4, 2018, entitled "Systems, Apparatus, and Methods for Placing a Gastrostomy Tube".

### Background

Embodiments described herein relate to systems, apparatus, and methods for delivering a medical device beyond a bifurcation in a body lumen. For example, embodiments described herein relate to systems, apparatus, and methods for translating a tube through an esophagus of the patient to a stomach of the patient.

Delivering a medical device to a location within a patient may include guiding the medical device through a bifurcated body lumen. Such procedures are sometimes performed blindly, risking misplacement of the medical device within the patient's body and/or injury to the patient's body. Furthermore, delivery of a medical device through a lumen of a patient can be even more difficult in high risk patients (e.g., sedated patients, endotracheally-intubated patients, and/or agitated patients). Additionally, while the position of a medical device within the patient can be confirmed via X-ray imaging, X-ray imaging carries the risk of radiation-induced injuries.

Thus, there is a need for systems, apparatus, and methods of delivering a medical device beyond a bifurcation in a body lumen which reduce risks to the patient and allow for the medical device to be quickly and easily placed in its intended location. US2003/040671 discloses a medical tube for insertion into the body of a patient. The medical tube includes a tube or device suitable for insertion into the patient's body, and a permanent magnet associated therewith. The magnet may be solid or non-solid, and may be rigid or non-rigid. In one embodiment, the magnet is hollow and associated with the medical tube such that the tube may be used for its intended purposes. In another embodiment, the magnet is solid and, after insertion into the body of the patient, is displaced such that it does not interfere with the intended use of the medical tube. In a further embodiment, the magnet is removable after placement of the medical tube.

### Summary

Systems, apparatus, and methods for placing an elongated tube within a patient's body are described herein. The invention is defined by the appended claims and discloses an apparatus comprising an elongated tube, a magnetic tip, and an inflatable member. The elongated tube has a first end and a second end, and defines a first lumen and a second lumen. The first lumen extends from the first end to the second end. The magnetic tip is coupled to the first end of the elongated tube. The magnetic tip is tapered toward a distal end of the magnetic tip. An inflatable member is disposed within the elongated tube proximal of the tapered magnetic tip and fluidically coupled to the second lumen such that the inflatable member can receive fluid via the second lumen.

### Brief Description of the Drawings

FIG. 1 is a schematic illustration of a portion of a patient, according to an embodiment.
FIG. 2 is a schematic illustration of a tube disposed in a patient, according to an embodiment.
FIG. 3 is a schematic illustration of a system, according to an embodiment.
FIGS. 4A-4E illustrate a system and use of the system, according to an embodiment.
FIGS. 5A-5D illustrate a system and use of the system, according to an embodiment.
FIG. 6 is an exploded perspective view of a distal subassembly, according to an embodiment.
FIG. 7 is a perspective view of an external magnetic member and a strap assembly, according to an embodiment.
FIG. 8 is a perspective view of an assembly, according to an embodiment.
FIG. 9 is an example of an ultrasound image of a distal end of a magnetically-responsive assembly, such as any of the assemblies described herein, within a patient, according to an embodiment.
FIG. 10 is a flow chart illustrating a method of using any of the systems and/or assemblies described herein, according to an embodiment.
FIG. 11 is a flow chart illustrating a method of using any of the systems and/or assemblies described herein, according to an embodiment.

### Detailed Description

According to the invention, an apparatus includes an elongated tube, a magnetic tip, and an inflatable member. The elongated tube has a first end and a second end, and defines a first lumen and a second lumen. The first lumen extends from the first end to the second end. The magnetic tip is coupled to the first end of the elongated tube. The magnetic tip is tapered toward a distal end of the magnet tip. An inflatable member is disposed within the elongated tube proximal of the tapered magnetic tip and fluidically coupled to the second lumen such that the inflatable member can receive fluid via the second lumen.

In some embodiments, a system includes a magnetically responsive assembly and a magnetic member. The magnetically responsive assembly can include an elongated tube having a first end and a second end. The elongated tube can define a first lumen and a second lumen. The first lumen can extend from the first end to the second end. A magnetic tip generating a magnetic field can be coupled to the first end of the elongated tube. The magnetic tip can be tapered toward a distal end of the magnetic tip. The inflatable member can be disposed within the elongated tube proximal of the magnetic tip and fluidically coupled to the second lumen such that the inflatable member can receive fluid via the second lumen. The magnetic member can be configured to be disposed adjacent a surface of a patient and to apply a magnetic field such that, as the magnetic tip of the magnetically responsive assembly is translated through a first passageway toward an internal junction of the patient in which the first passageway, a second passageway, and a third passageway intersect, a magnetic force produced by the interaction of the magnetic field of the magnetic member and a magnetic field of the magnetic tip can urge the position of the magnetic tip to align the magnetic tip with the second passageway.

In some embodiments, a method includes introducing a magnetic tip of an elongated tube into an oropharynx region of a patient via one of a nasal cavity or an oral cavity of the patient. The magnetic tip can be tapered toward a distal end of the magnet tip. An external magnetic member can be applied to an external surface of the patient at a location corresponding to an internal junction of a hypopharynx of the patient and a larynx of the patient such that the external magnetic member applies a magnetic field to the internal junction. The magnetic tip can be translated through the hypopharynx, the magnetic field of the external magnetic member urging the magnetic tip toward a posterior wall of the hypopharynx as the magnetic tip is translated through the hypopharynx. The magnetic tip can be advanced through an esophagus of the patient to the stomach of the patient.

In some embodiments, a method includes disposing an external magnetic member on an external surface of a patient at a location corresponding to an internal junction of a first passageway, a second passageway, and a third passageway of the patient such that a first pole of the external magnetic member is oriented toward the external surface of the patient and a second pole of the external magnetic member is oriented away from the first pole such that the external magnetic member applies a magnetic field to the internal junction. The second pole can have an opposite polarity to the first pole. A magnetic tip of an elongated tube can be translated through an orifice of the patient, through the first passageway, through the internal junction, and into the second passageway, the magnetic tip being tapered toward a distal end of the magnet tip, the magnetic tip having a first pole oriented toward the external magnetic member and a second pole oriented away from the external magnetic member such that, as the magnetic tip is translated through the first passageway and the internal junction, the interaction of the first pole of the external magnetic member and the first pole of the magnetic tip shifts the magnetic tip into alignment with the second passageway.

Systems, apparatus, and methods described herein include the delivery of a medical device to locations within a patient's body. The medical device may include, for example, a tube such as a gastrostomy tube, a nasogastric tube, and/or a nasojejunal tube. The medical device may need to navigate or be guided beyond a bifurcation in a body lumen of the patient's body such that the medical device is advanced through an intended passageway and avoids an unintended route. Bifurcated body lumens may be encountered, for example, when a medical device is delivered to a patient's esophagus, stomach, trachea, or a particular lung.

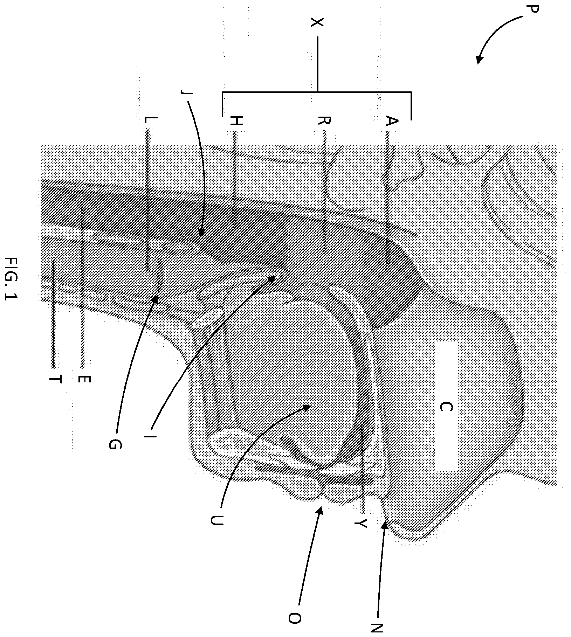

For example, an end of a tube may be delivered to a patient's stomach such that fluids can be delivered to the patient's stomach through the tube. FIG. 1 is a schematic illustration of a portion of a patient P. The patient P has an oral cavity Y, a nasal cavity C, a pharynx X. The patient P includes a nasal orifice N that provides access to the nasal cavity C and an oral orifice O that provides access to the oral cavity Y. The pharynx X includes a nasopharynx A, an oropharynx R, and a hypopharynx H. The patient P includes a junction J at which the pharynx X is coupled to a larynx L and an esophagus E. Specifically, the hypopharynx H is coupled to the larynx L at the junction J. The larynx L is connected to the trachea T. The patient P includes an epiglottis I, which is a flap of cartilage at the root of the tongue U of the patient P, which is depressed during swallowing to cover the opening to the larynx L. The larynx L also includes a glottis G, which is a slit-like opening between vocal cords of the patient P. Thus, a first pathway can include the nasal orifice N and nasal cavity C (or the oral orifice O and oral cavity Y), the nasopharynx A, the oropharynx R, and a first portion of the hypopharynx H. A second pathway can include a second portion of the hypopharynx H and the esophagus E. A third pathway can include the trachea T.

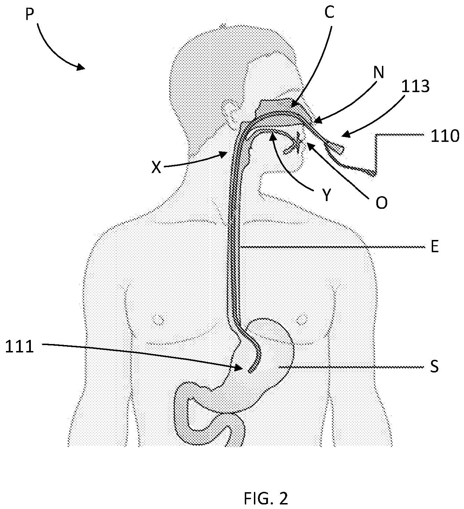

When a patient has difficulty swallowing food and/or liquid, a tube (e.g., a nasogastric tube) can be placed via an oral or nasal orifice and an esophagus of the patient such that nutrition can be provided directly through the tube to the stomach. FIG. 2 is a schematic illustration of a patient P having a nasal orifice N, a nasal cavity C, a pharynx X, an esophagus E, and a stomach S. As shown in FIG. 2, an elongated tube 110 (e.g., a nasogastric tube) can be inserted through the nasal orifice N, through the nasal cavity C, through the pharynx X, through the esophagus E and into the stomach S. Thus, the elongated tube 110 can have a first end 111 disposed within the stomach and a second end 113 disposed outside of the patient P, with the elongated tube 110 passing through the patient's nasal orifice N. While the elongated tube 110 is shown as being disposed within the patient's nasal orifice N and nasal cavity C, in some embodiments the elongated tube 110 can be inserted via an oral orifice O of the patient, into the patient's oral cavity Y, into the pharynx X, through the esophagus E, and into the stomach S. Thus, the stomach S can be accessible from the nasal orifice N or the oral orifice O via the pharynx X and esophagus E.

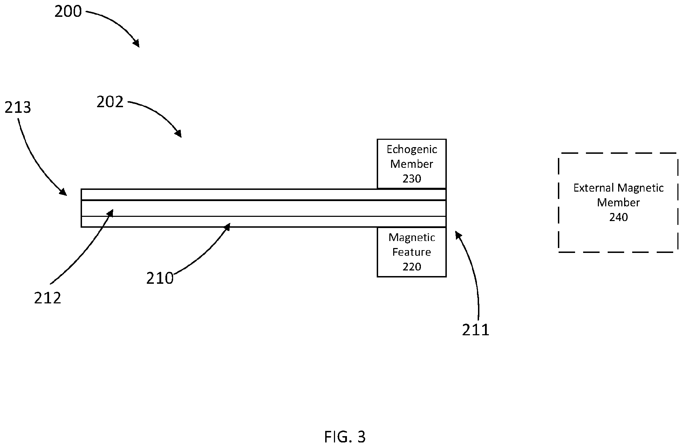

Navigation of the elongated tube through the patient, however, can be challenging and pose risks to the patient. If the elongated tube is misplaced within the patient's body, such as within a patient's larynx, the elongated tube can injure the patient. For example, the elongated tube can cause pulmonary damage, pneumothorax, and/or hemodynamic collapse. In some embodiments, however, an elongated tube can include features to aid in navigating the first end of the elongated tube to the stomach of the patient. For example, FIG. 3 is a schematic representation of a system 200. The system 200 includes an assembly 202. The assembly 202 includes an elongated tube 210, a magnetic feature 220, and an echogenic member 230. The elongated tube 210 can have a first end 211, a second end 213, and can define a feeding lumen 212. In some embodiments, the system can optionally include an external magnetic member 240.

In some embodiments, the first end 211 of the elongated tube 210 can be open such that fluid can flow through the second end 213, through the feeding lumen 212, through the first end 211, and into the stomach. In some embodiments, the elongated tube 210 can define a number of fenestrations in a sidewall of the elongated tube 210 near the first end 211 such that fluid can flow through the second end 213, through the feeding lumen 212, out of the fenestrations, and into the stomach. In some embodiments, a cap or cover can be coupled to the first end 211 such that fluid communication between the feeding lumen 212 and the stomach is only via the one or more side openings. In some embodiments, the second end 213 is coupled to and/or includes a sump or feeding port.

In some embodiments, the magnetic feature 220 can include a magnetic tip of the elongated tube 210. The magnetic feature 220 can be configured to apply a magnetic field to at least a portion of the patient's body. The magnetic tip can be coupled to the first end 211 of the elongated tube 210 and extend axially away from the first end 211 of the elongated tube 210. In some embodiments, the magnetic tip can have a first end opposite a second end, the second end can be coupled to the first end 211 of the elongated tube 210, and the magnetic tip can be tapered toward a first end of the magnetic tip. In some embodiments, the magnetic tip can be spatula-shaped. For example, the magnetic tip can include oppositely-disposed sidewalls and a bottom surface, the sidewalls tapered toward the bottom surface (e.g., relative to an axis coaxial with the first end 211 of the elongated tube 210). In some embodiments, the oppositely-disposed sidewalls can each additionally or alternatively include at least a portion extending diagonally relative to (e.g., tapering toward) the axis coaxial with the first end 211 of the elongated tube 210. For example, the magnetic tip can include a distal end having a straight edge disposed perpendicular to the coaxial axis, and the sidewalls of the magnetic tip can including portions that extend parallel to the coaxial axis and portions that taper diagonally from the parallel-extending portions to the straight edge. In some embodiments, the distal end of the magnetic tip may be curved from a first sidewall of the oppositely-disposed sidewalls to a second sidewall of the oppositely-disposed sidewalls.

In some embodiments, the magnetic tip can include an opening such that the feeding lumen 212 can be in fluidic communication with the stomach via the opening in the magnetic tip when the first end 221 of the elongated tube 210 is disposed within the stomach. In some embodiments, the magnetic tip can include a cap coupled to the first end 211 of the elongated tube 210, one or more magnetic components disposed within the cap. In some embodiments, the magnetic feature 220 can include one or more magnetic components disposed within the elongated tube 210. For example, the elongated tube 210 can include an end cap coupled to the first end 211, and one or more magnetic components can be enclosed within the end cap and/or the elongated tube 210. For example, in some embodiments, one or more spherical magnetic components (e.g., one, two, three, four, or more) can be disposed within the elongated tube 210 and/or an end cap coupled to the first end 211 of the elongated tube 210.

In some embodiments, rather than including a tapered or spatula-shaped magnetic tip as described above, a non-magnetic tapered or spatula-shaped tip can be coupled to the first end 211 of the elongated tube 210 and a magnetic feature can be disposed on and/or within the elongated tube 210.

In some embodiments, the magnetic feature 220 (e.g., a magnetic tip) can be formed of any suitable type of magnet. For example, the magnetic feature 220 can include a permanent magnet, such as a neodymium iron boron (NdFeB) magnet, a samarium cobalt (SmCo) magnet, an aluminum nickel cobalt (AlNiCo) magnet, a ceramic magnet, a ferrite magnet, and/or any other suitable rare earth magnet. In some embodiments, the magnetic feature 220 can include a temporary magnet. In some embodiments, the magnetic feature 220 can be an electromagnet, such as a solenoid. In some embodiments, the magnetic feature 220 can generate a magnetic field having an orientation (i.e., north (N) and south (S) poles). In other embodiments, the magnetic feature 220 can be formed of a ferromagnetic material that is not magnetized, i.e. does not generate its own magnetic field, but can be affected by an externally-applied magnetic field. For example, the magnetic feature 220 can be formed of iron, and application of an external magnetic field can attract the iron toward the source of the field, applying a force to the magnetic feature 220.

The echogenic member 230 is configured to be visualized via ultrasound such that the location of the echogenic member 230 (and, thus, the first end 211 of the elongated tube 210) within a patient can be verified. In some embodiments, the echogenic member 230 can be an inflatable member, such as an inflatable balloon. The system 200 can include an inflation lumen (not shown). In some embodiments, the inflation lumen can be separate from the elongated tube 210. In some embodiments, the inflation lumen can be defined by the elongated tube 210. For example, a central axis of the inflation lumen can extend parallel to a central axis of the feeding lumen 212. In some embodiments, the inflation lumen can include an inflation port on an end opposite the echogenic member 230. In some embodiments, the echogenic member 230 can be disposed on the elongated tube 210 such that the echogenic member 230 surrounds the elongated tube 210. In some embodiments, the echogenic member 230 can extend laterally from a side of the elongated tube 210 such that echogenic member extends asymmetrically relative to a central axis of the elongated tube 210. In some embodiments, the echogenic member 230 is configured to transition between an uninflated configuration and an inflated configuration (e.g., due to echogenic fluid being introduced into an interior of the echogenic member 230), the echogenic member 230 extending to a greater lateral extent relative to a centerline of the elongated tube 210 in the inflated configuration. In some embodiments, the echogenic member 230 does not extend beyond an outer surface of the elongated tube 210 such that the assembly 213 has a smooth outer profile with a substantially continuous outer diameter through the portions including the elongated tube 210 and the echogenic member 230. In some embodiments, rather than having a separate echogenic member 230, a portion of the elongated tube 210, a cap coupled to the first end 211 of the elongated tube 210, and/or the magnetic feature 220 can have echogenic properties such that the assembly 202 can be visualized using ultrasound.

The external magnetic member 240 can include any suitable magnet configured to apply a magnetic field to at least a portion of a patient's body that can interact with the magnetic field generated by the magnetic feature 220 to generate a force on the magnetic feature 220. The external magnetic member 240 can include, for example, a permanent magnet, such as a neodymium iron boron (NdFeB) magnet, a samarium cobalt (SmCo) magnet, an aluminum nickel cobalt (AlNiCo) magnet, a ceramic magnet, a ferrite magnet, and/or any other suitable rare earth magnet. In some embodiments, the magnetic feature 220 can be an electromagnet, such as a solenoid. In some embodiments, the magnetic feature 220 can generate a magnetic field having an orientation (i.e., north (N) and south (S) poles). In some embodiments, the external magnetic member 240 can be disposed within a strap assembly (not shown). The strap assembly can be configured to secure the external magnetic member 240 to a patient's body (e.g., a patient's neck).

In some embodiments, the external magnetic member 240 can be configured to be disposed on the surface of a patient's skin and can apply a magnetic field to at least a portion of a patient's body that can interact with the magnetic field generated by the magnetic feature 220 through various body tissue and organs disposed between the external magnetic member 240 on the surface of the patient's skin the magnetic feature 220 within the patient's body and across any suitable distance (e.g., about 10 cm, about 15 cm, and/or about 20 cm). For example, the external magnetic member 240 can be configured to be disposed on an anterior and/or posterior portion of a patient's neck. The interaction between the magnetic field generated by the external magnetic member 240 and the magnetic field generated by the magnetic feature 220 can produce a magnetic force that urges the position of the magnetic feature 220 in a direction toward or away from the external magnetic member 240, and thus also urges the first end 211 of the elongated tube 210 relative to the external magnetic member 240. In some embodiments, the external magnetic member 240 can have a first side and a second side, the second side disposed opposite of the first side. The external magnetic member 240 can have a first pole oriented in the direction the first side faces and a second pole oriented in the direction the second side faces, the first pole having an opposite polarity to the second pole. In some embodiments, the external magnetic member 240 can be configured to attract the magnetic feature 220 toward the external magnetic member 240 (e.g., if the external magnetic member 240 is disposed near the magnetic feature 220 with a first pole applying a magnetic force to the magnetic feature 220 of an opposite polarity as the magnetic feature 220 facing the external magnetic member 240). In some embodiments, the external magnetic member 240 can be configured to repel the magnetic feature 220 away from the external magnetic member 240 (e.g., if the external magnetic member 240 is disposed near the magnetic feature 220 with a first pole applying a magnetic force to the magnetic feature 220 of the same polarity as the magnetic feature 220 facing the external magnetic member 240). In some embodiments, the magnetic feature 220 (e.g., a magnetic tip) can be oriented such that the magnetic feature 220 can apply a force (e.g., a magnetic force) in a first direction lateral to a central axis of the elongated tube 210, and the external magnetic member 240 can be oppositely polarized and disposed a distance from the magnetic feature 220 along the first direction such that the magnetic tip is moved in a second direction opposite the first direction. In some embodiments, the external magnetic member 240 can be arranged in a first configuration relative to the magnetic feature 220 (e.g., near a first side of the magnetic feature 220) such that the a first pole (e.g., N) of the external magnetic member 240 faces a pole of the magnetic feature 220 having the same polarity and, thus, applies a repulsive force to the magnetic feature 220. The external magnetic member 240 can be arranged in a second configuration relative to the magnetic feature 220 (e.g., near a second side opposite the first side) such that the first pole of the external magnetic member 240 faces a pole of the magnetic feature 220 having an opposite polarity (e.g., S), and thus, applies an attractive force to the magnetic feature 220.

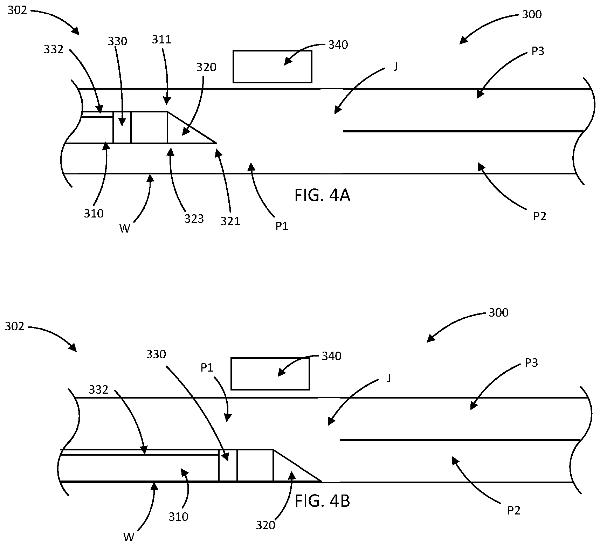

FIGS. 4A-4E illustrate a system 300 and a method of using the system 300 to navigate an internal junction of a patient. The system 300 can be the same or similar in structure and/or function to any of the systems described herein, such as the system 200. For example, as shown in FIG. 4A, the system 300 includes a magnetically responsive assembly 302 and an external magnetic member 340. The assembly 302 includes an elongated tube 310 (e.g., a nasogastric tube), a magnetic tip 320, and an echogenic member 330. Each of the external magnetic member 340 and the magnetic tip 320 can be configured to apply a magnetic field to at least a portion of the patient's body. The elongated tube 310 can include a first end 311, a second end opposite the first end 311, and a feeding lumen. As shown, the magnetic tip 320 can be coupled to the first end 311 of the elongated tube 310 and can be tapered. For example, the magnetic tip 320 can have a first end 321 opposite a second end 323. The second end 323 of the magnetic tip 320 can be coupled to the first end 311 of the elongated tube 310, and the magnetic tip 320 can be tapered toward a first end 321 of the magnetic tip 320. In some embodiments, the magnetic tip 320 can be spatula-shaped. For example, the magnetic tip 320 can include oppositely-disposed sidewalls and a bottom surface, the sidewalls tapered toward the bottom surface (e.g., relative to an axis coaxial with a central axis of the first end 311 of the elongated tube 310). In some embodiments, the oppositely-disposed sidewalls can each additionally or alternatively include at least a portion extending diagonally relative to (e.g., tapering toward) the axis coaxial with the first end 311 of the elongated tube 310. For example, the magnetic tip 320 can include a distal end having a straight edge disposed perpendicular to the coaxial axis, and the sidewalls of the magnetic tip 320 can including portions that extend parallel to the coaxial axis and portions that taper diagonally from the parallel-extending portions to the straight edge. In some embodiments, the distal end of the magnetic tip may be curved from a first sidewall of the oppositely-disposed sidewalls to a second sidewall of the oppositely-disposed sidewalls. In some embodiments, the magnetic tip 320 can include an opening such that the feeding lumen can be in fluidic communication with the stomach via the opening in the magnetic tip 320 when the first end 321 of the elongated tube 310 is disposed within the stomach. The system 300 can also include an inflation lumen 332 such that the echogenic member 330 can be filled with echogenic fluid via the inflation lumen 332.

As shown in FIG. 4A, the assembly 302 can be disposed within a first passageway P1 of a patient. The first passageway P1 can be, for example, at least a portion of a pharynx of the patient. The assembly 302 can be translated into the first passageway P1 of the patient via, for example, an oral orifice or nasal orifice of the patient. The external magnetic member 340 can be disposed on a surface of the patient (e.g., on the patient's skin) near an internal junction J of the patient. The first passageway P1 can be coupled to a second passageway P2 and a third passageway P3 at the internal junction J such that the internal junction J forms the intersection of the first passageway P1, the second passageway P2, and the third passageway P3. The second passageway P2 can be, for example, a larynx of the patient. The third passageway P3 can be, for example, an esophagus of the patient. In some embodiments, the first passageway P1 can be a first portion of a hypopharynx of the patient, the second passageway P2 can be a second portion of the hypopharynx, the second portion of the hypopharynx coupled to an esophagus of the patient, and the third portion P3 can be a larynx of the patient.

The external magnetic member 340 can be aligned with the internal junction J and/or with a portion of the first passageway P1 near the internal junction J. In some embodiments, the external magnetic member 340 and the magnetic tip 320 can be arranged in the same plane as a central axis of the first passageway P1, a central axis of the second passageway P2, and a central axis of the third passageway P3. In some embodiments, the external magnetic member 340 has a first pole oriented toward the surface of the patient and a second pole oriented away from the first pole, the second pole having an opposite polarity to the first pole.

As shown in FIG. 4B, the assembly 302 can be translated toward the internal junction J. The external magnetic member 340 can be configured and disposed such that, as the magnetic tip 320 is translated through the first passageway P1 toward the internal junction J, the external magnetic member 340 can apply a magnetic field to the magnetic tip 320 to urge or shift the position of the magnetic tip 320 into alignment with the second passageway. Specifically, a magnetic force can be produced by the interaction of the magnetic field generated by the external magnetic member 340 and the magnetic field generated by the magnetic tip 320 that can urge or shift the position of the magnetic tip 320. The magnetic tip 320 can have a first pole oriented toward the external magnetic member 340 and a second pole oriented away from the external magnetic member 340 such that, as the magnetic tip 320 is translated through the first passageway P1 and the internal junction J, the interaction of the first pole of the external magnetic member 340 and the first pole of the magnetic tip 320 shifts the magnetic tip into alignment with the second passageway P2. In some embodiments, the first pole of the magnetic tip 320 has the same polarity as the first pole of the external magnetic member 340 such that the magnetic tip 320 is repelled toward a wall W of the first passageway P1 by the external magnetic member 340 as the magnetic tip 320 is translated through the first passageway P1 and the second passageway P2. For example, as shown in FIG. 4B, the magnetic tip 320 can be urged against the wall W of the first passageway P1, the wall W disposed opposite the position of the external magnetic member 340. In some embodiments, the external magnetic member 340 can be configured to maintain the magnetic tip 320 in contact with a posterior hypopharynx wall of the patient as the magnetic tip 320 is translated through the hypopharynx and into the esophagus of the patient.

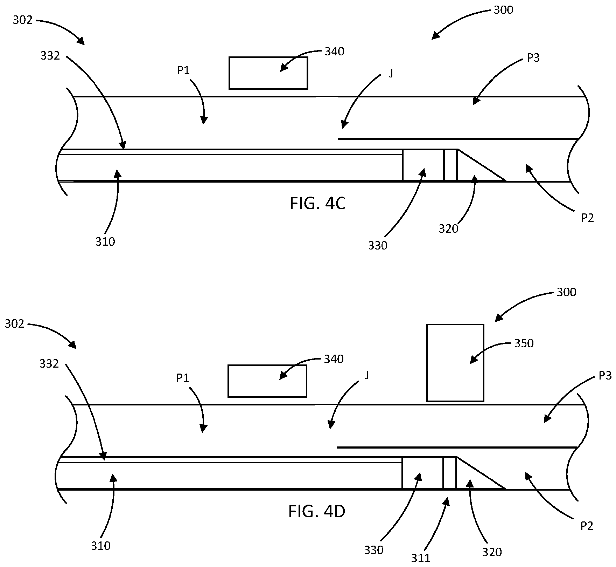

As shown in FIG. 4C, with the magnetic tip 320 aligned with the second passageway P2, the assembly 302 can be further translated relative to the internal junction J such that the magnetic tip 320 and/or the first end 311 of the assembly 302 is disposed within the second passageway P2. In some embodiments, the tapered shape of the magnetic tip 320 can assist in ensuring that the magnetic tip 320 is translated into the second passageway P2 rather than the third passageway P3. For example, if the magnetic tip 320 and the second passageway P2 are only partially aligned by the interaction between the external magnetic member 340 and the magnetic tip 320 prior to advancing the assembly 302 past the internal junction J, if the distal-most end (e.g., the portion of the magnetic tip 320 having the greatest length) is aligned with the second passageway P2 side of the internal junction J, the assembly 302 can be translated into the second passageway P2 by advancing the assembly 302 such that the tapered portion of the magnetic tip 320 engages a sidewall of the second passageway P2 and the translation of the magnetic tip 320 relative to the sidewall urges the magnetic tip 320 and the first end 311 of the elongated tube 310 into the second passageway P2.

As shown in FIG. 4D, an ultrasound probe 350 can be used to verify the location of the first end 311 of the assembly 302 within the patient. Specifically, the ultrasound probe 350 can be used to verify that the assembly 302 is disposed in an intended passageway (e.g., the second passageway P2) rather than an unintended passageway (e.g., the third passageway P3). As shown in FIG. 4D, after advancing the first end 311 of the elongated tube 310 relative to the internal junction J, the ultrasound probe 350 can be applied to the surface of the patient such that the location of the echogenic member 330 can be identified. In some embodiments, fluid can be delivered to the echogenic member 330 via the inflation lumen 332 such that the echogenic member 330 can be visualized via the ultrasound probe 350. If the echogenic member 330 is verified as being disposed within the intended passageway (e.g., the second passageway P2), the first end 311 of the elongated tube 310 can be further advanced (e.g., through the esophagus and into the stomach of the patient). If the echogenic member 330 is visualized as being in an unintended passageway (e.g., the third passageway P3), the assembly 302 can be withdrawn such that the magnetic tip 320 and the first end 311 of the elongated tube 310 are disposed in the first passageway P1. In some embodiments, fluid can be removed from the echogenic member 330 prior to withdrawing the magnetic tip 320 and the first end 311 of the elongated tube 310. The external magnetic member 340 and the magnetic tip 320 can then be manipulated (e.g., via moving the external magnetic member 340 and/or the magnetic tip 320) to realign the magnetic tip 320 with the intended passageway (e.g. the second passageway). The assembly 302 can then be advanced again relative to the internal junction J (e.g., into the second passageway P2) and the position of the echogenic member 330 can be verified using the ultrasound probe 350 (e.g., fluid can be again introduced to the echogenic member 330 and viewed via the ultrasound probe 350). If the echogenic member 330 is in the intended passageway (e.g., the second passageway P2), the first end 311 of the elongated tube 310 can be advanced (e.g., to the stomach). In some embodiments, the external magnetic member 340 and the magnetic tip 320 can be arranged such that a line passing through both the external magnetic member 340 and the magnetic tip 320 can intersect a central axis of the second passageway P2 and a central axis of the third passageway P3 when the magnetic tip is position in the second passageway P2.

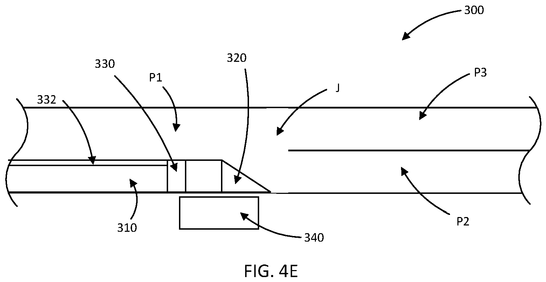

Although the external magnetic member 340 is shown as being disposed on a side of the patient corresponding to the third passageway P3 (e.g., an anterior surface of the patient such as the front side of the patient's neck), in some embodiments, the external magnetic member 340 can be disposed on a side of the patient corresponding to the second passageway P2 (e.g., a posterior surface of the patient such as the back of the patient's neck). For example, as shown in FIG. 4E, which is an alternative arrangement of the system 300, the external magnetic member 340 can be positioned on the side of the patient closer to the second passageway P2 than the third passageway P3 near the internal junction J. Thus, rather than repelling the magnetic tip 320 toward the posterior side of the first passageway P1, the external magnetic member 340 can attract the magnetic tip 320 toward the posterior side of the first passageway P1. For example, the external magnetic member 340 can be positioned on a surface of the patient (e.g., the skin) such that the second pole is oriented toward the magnetic tip 320 and the first pole is oriented away from the magnetic tip 320 and such that the magnetic tip 320 is attracted toward the second pole of the external magnetic member 340.

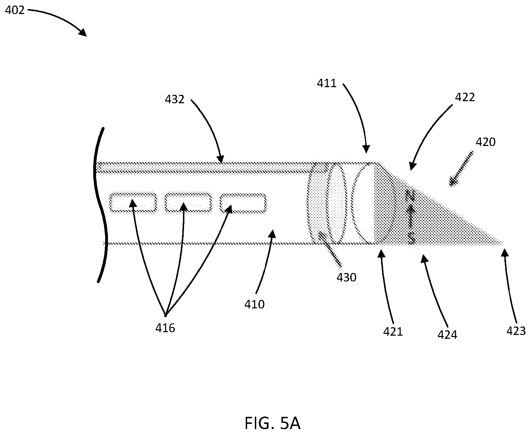

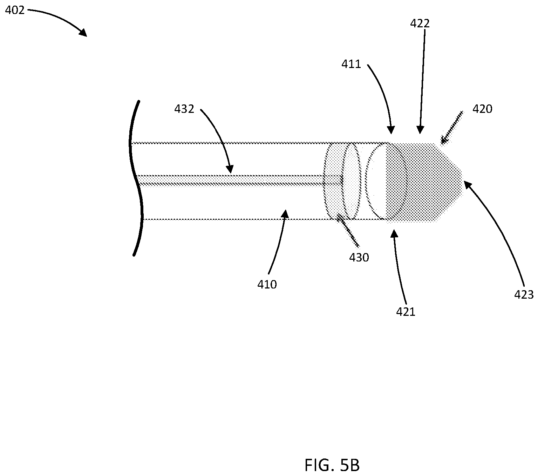

FIGS. 5A-5D illustrate a portion of a system 400 and a method of using the system 400 to navigate an internal junction of a patient. The system 400 can be the same or similar in structure and/or function to any of the systems described herein, such as the system 300 and/or the system 400. For example, as shown in FIGS. 5A and 5B, the system 400 includes a magnetically responsive assembly 402. FIGS. 5A and 5B are a schematic illustration of a side view and a top view, respectively, of the magnetically responsive assembly 402. The assembly 402 includes an elongated tube 410, a magnetic tip 420, and an echogenic member 430. The elongated tube 410 can include a first end 411 and a second end (not shown) opposite the first end 411. The elongated tube 410 can define a feeding lumen. The elongated tube 410 can define a number of fenestrations 416 such that the feeding lumen of the elongated tube 410 can be in fluid communication with a stomach of the patient via the fenestrations 416. The feeding lumen can extend from the second end to the fenestrations 416 and/or the first end 411. Although FIG. 5A shows the elongated tube 410 as defining three fenestrations 416, the elongated tube 410 can define any suitable number of fenestrations. The system 400 can include an inflation lumen 432 such that the echogenic member 430 can be filled with echogenic fluid via the inflation lumen 432.

As shown in FIGS. 5A and 5B, the magnetic tip 420 can be coupled to a first end 411 of the elongated tube 410 and can be tapered. For example, the magnetic tip 420 can have a first end 421 opposite a second end 423. The second end 423 of the magnetic tip 420 can be coupled to the first end 411 of the elongated tube 410, and the magnetic tip 420 can be tapered toward a first end 421 of the magnetic tip 420. In some embodiments, the magnetic tip 420 can be spatula-shaped. For example, the magnetic tip 420 can include oppositely-disposed sidewalls and a bottom surface, the sidewalls tapered toward the bottom surface (e.g., relative to an axis coaxial with a central axis of the first end 411 of the elongated tube 410). In some embodiments, the oppositely-disposed sidewalls can each additionally or alternatively include at least a portion extending diagonally relative to (e.g., tapering toward) the central axis of the first end 411 of the elongated tube 410. For example, the second end 423 of the magnetic tip 420 can include a straight edge disposed perpendicular to the central axis, and the sidewalls of the magnetic tip 420 can including portions that extend parallel to the central axis and portions that taper diagonally from the parallel-extending portions to the straight edge. In some embodiments, the second end 423 of the magnetic tip may be curved from a first sidewall of the oppositely-disposed sidewalls to a second sidewall of the oppositely-disposed sidewalls. Additionally, as shown in FIG. 5A, the magnetic tip 420 can have a first side 422 and a second side 424, the first side 422 having an opposite polarity from the second side 424. In some embodiments, the magnetic tip 420 can include an opening such that the feeding lumen of the elongated tube 410 can be in fluidic communication with the stomach via the opening in the magnetic tip 420 when the first end 421 of the elongated tube 410 is disposed within the stomach. The magnetic tip 420 can be configured to apply a magnetic field to at least a portion of the patient's body.

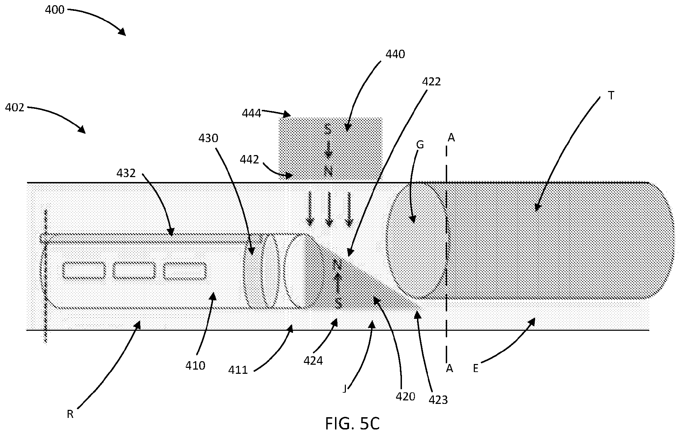

As shown in FIG. 5C, a portion of the assembly 402 can be disposed within a superior portion of the oropharynx R of a patient. The first end 411 of the assembly 402 can be translated into the superior portion of the oropharynx R of the patient via, for example, an oral orifice or nasal orifice of the patient. An external magnetic member 440, which can be the same or similar in structure and/or function to any of the external magnetic members described herein, can be disposed on a surface of the patient (e.g., on the patient's skin) near an internal junction J of the patient. For example, the external magnetic member 440 can be configured to apply a magnetic field to at least a portion of the patient's body. The trachea can be coupled to the superior portion of the oropharynx R via the glottis G. Similarly, the esophagus E can be coupled to the superior portion of the oropharynx R, for example, via an inferior portion of the oropharynx R.

As shown in FIG. 5C, the external magnetic member 440 can be aligned with the internal junction J and/or with a portion of the oropharynx R near the internal junction J. The external magnetic member 440 can have a first side 442 having a first pole and a second side 444 opposite the first side 442 having a second pole. Similarly, the magnetic tip 420 can have a first side 422 having a first pole and a second side 424 opposite the first side having a second pole. The first pole of the first side 442 of the external magnetic member 440 and the first pole of the first side 422 of the magnetic tip 420 can be the same polarity such that the external magnetic member 440 repels the magnetic tip 420 when the external magnetic member 440 and the magnetic tip 420 are arranged with the first side 422 of the magnetic tip 420 facing the first side 442 of the external magnetic member 440. Thus, when the assembly 402 is disposed such that the magnetic tip 420 is disposed within the superior portion of the oropharynx R, the external magnetic member 440 can be arranged on the anterior portion of the patient's neck such that the interaction of the magnetic field of the external magnetic member 440 and the magnetic field of the magnetic tip 420 produces a magnetic force to the magnetic tip 420 (e.g., the external magnetic member 440 repels the first side 422 of the magnetic tip 420), shifting the magnetic tip 420 and the first end 411 of the elongated tube 410 posteriorly. Although not shown, the external magnetic member 440 can alternatively be arranged on a posterior portion of the patient's neck with the first side 442 oriented toward the patient such that external magnetic member 440 attracts the second side 424 of the magnetic tip 420 posteriorly (the first side 442 of the external magnetic member 440 and the second side 424 of the magnetic tip 420 having opposite polarities).

With at least the distal-most end of the assembly 402 (e.g., the first end 423 of the magnetic tip 420) aligned with the inferior portion of the oropharynx R and/or the esophagus E, the assembly 402 can be translated into the inferior portion of the oropharynx R. With the first end 423 of the magnetic tip 410 outside of the trachea T, as shown in FIG. 5C, further advancement of the assembly 402 will urge the assembly 402 toward the esophagus E rather than through the glottis G and into the trachea T due to the tapered shape of the magnetic tip 410.

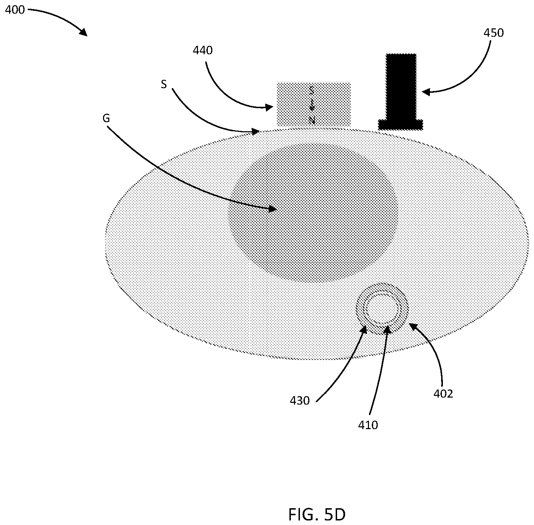

FIG. 5D is a schematic illustration of a cross-section of the patient taken along, for example, line A-A in FIG. 5C representing a cross-section of a patient at the junction of the patient's glottis G and trachea. As shown, the location of the assembly 402 can be verified relative to unintended pathways, such as a pathway including the patient's glottis G. As shown, the external magnetic member 440 can be disposed on a surface S of the patient while the location of the assembly 402 is verified using an ultrasound probe 450. After advancing the first end 411 of the elongated tube 410 relative to the internal junction J, the ultrasound probe 450 can be applied to the surface of the patient such that the location of the echogenic member 430 can be identified. Fluid can be delivered the echogenic member 430 via the inflation lumen 432 such that the echogenic member 430 can be visualized via the ultrasound probe 450. If the echogenic member 430 is verified as being disposed within the intended passageway (e.g., the inferior portion of the oropharynx R), the first end 411 of the elongated tube 410 can be further advanced (e.g., through the esophagus and into the stomach of the patient). If the echogenic member 430 is visualized as being in an unintended passageway (e.g., the trachea), the assembly 402 can be withdrawn such that the magnetic tip 420 and the first end 411 of the elongated tube 410 are disposed in the superior portion of the oropharynx R. The external magnetic member 440 and the magnetic tip 420 can then be manipulated (e.g., via moving the external magnetic member 440 and/or the magnetic tip 420) to realign the magnetic tip 420 with the intended passageway (e.g. the inferior portion of the oropharynx R). The assembly 402 can then be advanced again relative to the internal junction J (e.g., into the second passageway P2) and the position of the echogenic member 430 can be verified using the ultrasound probe 450. If the echogenic member 430 is in the intended passageway (e.g., the second passageway P2), the distal end 411 of the elongated tube 410 can be advanced (e.g., to the stomach).

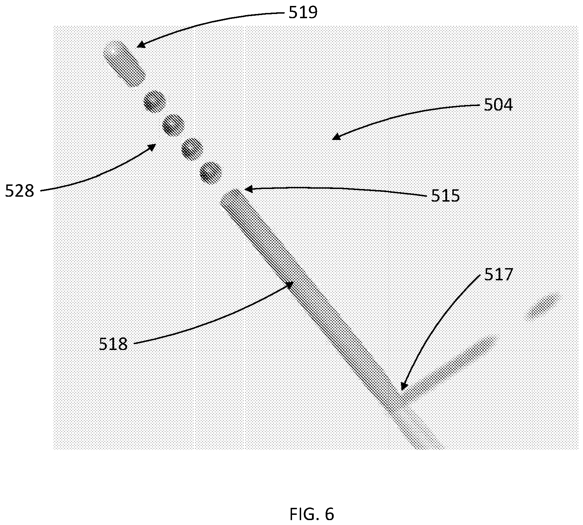

In some embodiments, a system can include a distal subassembly coupled to a first end of an elongated tube, the distal subassembly including a magnetic feature. For example, FIG. 6 is an exploded perspective view of a distal subassembly 504. The distal subassembly 504 can be coupled to the first end of any of the elongated tubes described herein. The distal subassembly 504 can include a tapered portion 518, a cap 519, and number of magnetic components 528. For example, as shown, the distal subassembly 504 can include four spherical magnetic components 528. In some embodiments, any suitable component of the distal subassembly 505 can be echogenic (e.g., be formed of an echogenic material or include an echogenic surface). For example, the tapered portion 518, the magnetic components 528, and/or the cap 519 can be echogenic. In some embodiments, the distal subassembly 504 can include an inflatable member which can be filled with echogenic fluid. In some embodiments, the spherical magnetic components 528 can be, for example, spherical 1/8" N52 neodymium magnets.

The tapered portion 518 can have a first end 515 and a second end 517. The second end 517 can be coupled to the first end of any of the elongated tubes described herein. The first end 515 can have a larger inner diameter and outer diameter than the inner diameter and outer diameter of the second end 517. For example, the first end 515 can have an inner diameter larger than the outer diameter of the magnetic components 528. The second end 517 can have an inner diameter smaller than the outer diameter of the magnetic components 528. Thus, the magnetic components can be prevented from traveling through the second end 517 and into an elongated tube coupled to the second end 517 due to the elongated tube being of a larger diameter than the opening defined at the second end 517. Additionally, the cap 519 can be coupled to the first end 515 such that the magnetic components 528 are enclosed within the cap 519 and/or the tapered portion 518.

The distal subassembly 504 can be used the same or similarly to any of the magnetic features described herein, such as magnetic feature 220, magnetic tip 320, and/or magnetic tip 420. For example, an external magnetic member (e.g., the external magnetic member 240) can be used to shift or urge the magnetic components 528 (and, thus, the distal subassembly 504) relative to various internal passageways of a patient. With the distal subassembly 504 aligned with an intended passageway (e.g. an inferior portion of an oropharynx), the distal subassembly 504 can be advanced into the intended passageway (e.g., via advancing an elongated tube such as any of the elongated tubes herein coupled to the distal subassembly 504).

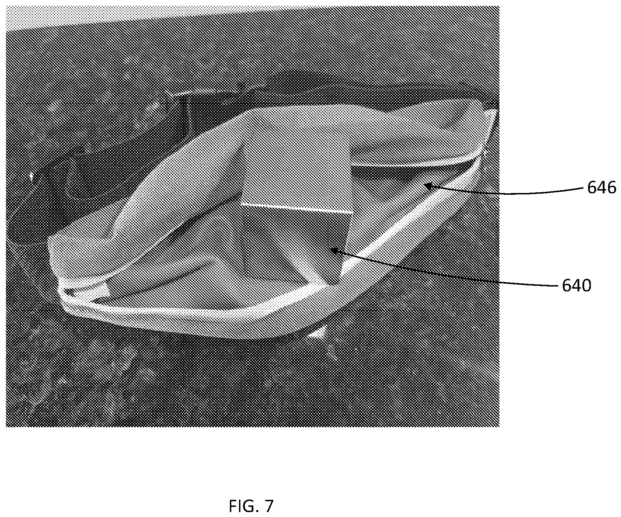

FIG. 7 is a perspective view of an external magnetic member 640 and a strap assembly 646. The external magnetic member 640 can be the same or similar to any of the external magnetic members described herein. The strap assembly 646 can receive the external magnetic member 640 in a pouch of the strap assembly 646. The external magnetic member 640 can be secured in the pouch of the strap assembly 646 using any suitable securement feature, such as, for example, a zipper or a hook-and-loop feature. In use, the strap assembly 646 can be coupled to a patient such that the external magnetic member 640 is held in place on the patient. For example, the strap assembly 646 can be coupled to a patient's neck such that the external magnetic member 640 is secured to an anterior skin portion or a posterior skin portion of the patient's neck. In some embodiments, the external magnetic member 640 can be, for example, a SuperMagnetMan 2" by 2" by 2" N52 Magnetic Cube. In some embodiments, the strap assembly 646 can be, for example, an expandable sports belt.

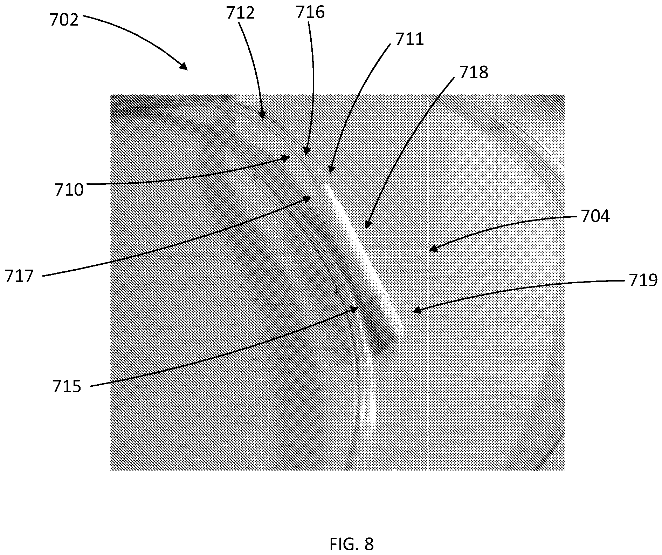

FIG. 8 is a perspective view of a portion of an assembly 702. The assembly 702 can be the same or similar in structure and/or function to any of the assemblies described herein. For example, the assembly 702 includes an elongated tube 710 and a distal subassembly 704. The elongated tube 710 can have a first end 711, a second end (not shown), and a feeding lumen 712. The elongated tube 710 can define a fenestration 716 in a sidewall of the elongated tube 710 such that the feeding lumen 712 can be in fluid communication with, for example, a stomach of a patient, via the fenestration 716. The distal subassembly 704 can include a tapered portion 718, a cap 719, and a number of magnetic components (not shown). For example, a number of spherical magnetic components can be enclosed within the tapered portion 718 and/or cap 719. In some embodiments, the spherical magnetic components can be, for example, spherical 1/8" N52 neodymium magnets. In some embodiments, any suitable member of the assembly 702 can be echogenic. For example, the tapered portion 718, the magnetic components, and/or the cap 719 can be echogenic. In some embodiments, the assembly 702 can include an inflatable member which can be filled with echogenic fluid.

As shown, the tapered portion 718 can have a first end 715 and a second end 717. The second end 717 can be coupled to the first end of any of the elongated tubes described herein. The first end 715 can have a larger inner diameter and outer diameter than the inner diameter and outer diameter of the second end 717. For example, the first end 715 can have an inner diameter larger than the outer diameter of the magnetic components. The second end 717 can have an inner diameter smaller than the outer diameter of the magnetic components. Thus, the magnetic components can be prevented from traveling through the second end 717 and into the elongated tube 710 due to the magnetic components having a larger diameter than the opening defined at the second end 717. Additionally, the cap 719 can be coupled to the first end 715 such that the magnetic components 728 are enclosed within the cap 719 and/or the tapered portion 718. In some embodiments, the tapered portion 718 can be formed as a tube that tapers from a 16 Fr tube size at the first end 715 to a 12 Fr tube size at the second end 717.

The distal subassembly 704 can be used the same or similarly to any of the magnetic features described herein, such as magnetic feature 220 and/or magnetic tip 320. For example, an external magnetic member (e.g., the external magnetic member 240) can be used to shift or urge the magnetic components 728 (and, thus, the distal subassembly 704) relative to various internal passageways of a patient. With the distal subassembly 704 aligned with an intended passageway (e.g. an inferior portion of an oropharynx), the distal subassembly 704 can be advanced into the intended passageway (e.g., via advancing the elongated tube 710 coupled to the distal subassembly 704).

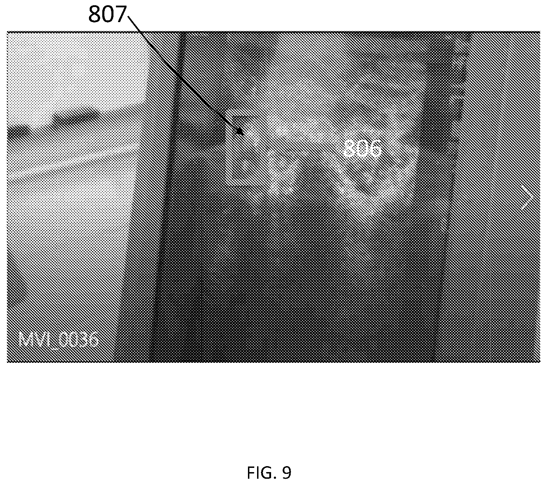

FIG. 9 is an example of an ultrasound image of a distal end of a magnetically-responsive assembly, such as any of the assemblies described herein, within a patient. Specifically, a portion 807 of an assembly can be seen in the area identified by box 806. The portion 807 can include, for example, an echogenic member, such as any of the echogenic members described herein.

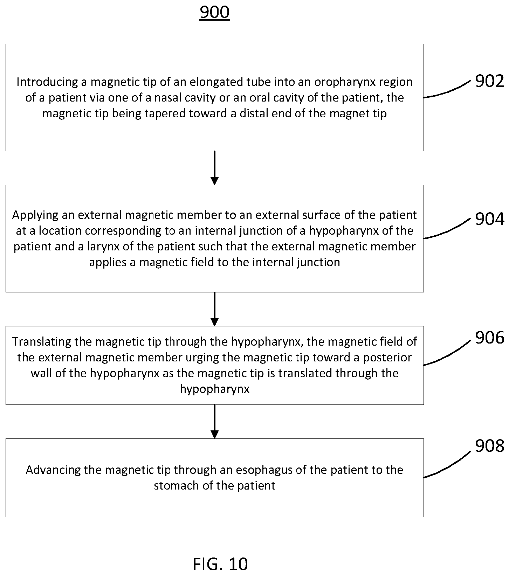

FIG. 10 is a flow chart illustrating a method 900 of using any of the systems and/or assemblies described herein. The method 900 includes introducing 902 a magnetic tip of an elongated tube into an oropharynx region of a patient via one of a nasal cavity or an oral cavity of the patient. The magnetic tip can be tapered toward a distal end of the magnet tip. An external magnetic member can be applied 904 to an external surface of the patient at a location corresponding to an internal junction of a hypopharynx of the patient and a larynx of the patient such that the external magnetic member applies a magnetic field to the internal junction. The magnetic tip can be translated 906 through the hypopharynx, the magnetic field of the external magnetic member urging the magnetic tip toward a posterior wall of the hypopharynx as the magnetic tip is translated through the hypopharynx. The magnetic tip can be advanced 908 through an esophagus of the patient to the stomach of the patient.

Optionally, prior to advancing the magnetic tip through the esophagus and to the stomach of the patient, an echogenic member or an echogenic portion coupled to the elongated tube can be visualized (e.g., via ultrasound) to verify the location of the magnetic tip within the patient. For example, an inflatable member (e.g., a balloon member) coupled to the elongated tube may be filled with an echogenic fluid and an ultrasound probe may be placed on a surface of the patient such that the inflatable member can be visualized using the ultrasound probe. If the echogenic member or echogenic portion is visualized in an intended location (e.g., within the esophagus), the magnetic tip can then be advanced to the stomach. If the echogenic member or echogenic portion is visualized in an unintended location (e.g., within a patient's larynx), the magnetic tip may be retracted or withdrawn a distance (e.g., to a location prior to the internal junction of the hypopharynx and the larynx), the external magnetic member may be repositioned relative to the patient, and the magnetic tip may be again translated beyond the internal junction of the patient, through the hypopharynx, and into the patient's esophagus. The echogenic member or echogenic portion may be visualized again to confirm the location of the magnetic tip within the hypopharynx beyond the internal junction prior to advancement into the patient's esophagus.

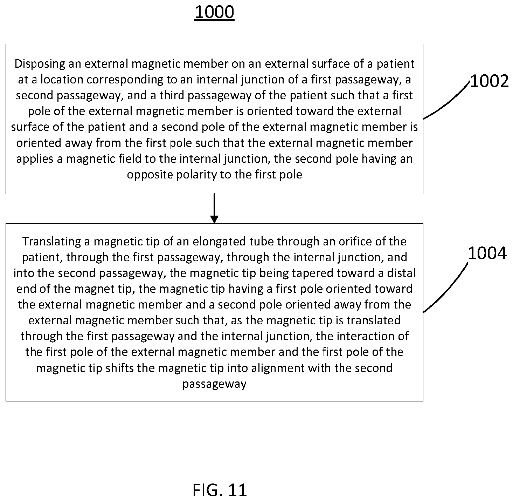

FIG. 11 is a flow chart illustrating a method 1000 of using any of the systems and/or assemblies described herein. The method 1000 can include disposing 1002 an external magnetic member on an external surface of a patient at a location corresponding to an internal junction of a first passageway, a second passageway, and a third passageway of the patient such that a first pole of the external magnetic member is oriented toward the external surface of the patient and a second pole of the external magnetic member is oriented away from the first pole such that the external magnetic member applies a magnetic field to the internal junction. The internal junction, the first passageway, and the second passageway may be any suitable internal junction, first passageway, and second passageway of a patient. The second pole can have an opposite polarity to the first pole. A magnetic tip of an elongated tube can be translated 1004 through an orifice of the patient, through the first passageway, through the internal junction, and into the second passageway. The magnetic tip can be tapered toward a distal end of the magnet tip. The magnetic tip can have a first pole oriented toward the external magnetic member and a second pole oriented away from the external magnetic member such that, as the magnetic tip is translated through the first passageway and the internal junction, the interaction of the first pole of the external magnetic member and the first pole of the magnetic tip shifts the magnetic tip into alignment with the second passageway.

Optionally, after advancing the magnetic tip through the internal junction and into the second passageway, an echogenic member or an echogenic portion coupled to the elongated tube can be visualized (e.g., via ultrasound) to verify the location of the magnetic tip within the patient. For example, an inflatable member (e.g., a balloon member) coupled to the elongated tube may be filled with an echogenic fluid and an ultrasound probe may be placed on a surface of the patient such that the inflatable member can be visualized using the ultrasound probe. If the echogenic member or echogenic portion is visualized in the second passageway, the magnetic tip can advanced farther beyond the internal junction. If the echogenic member or echogenic portion is visualized in an unintended location (e.g., within the first passageway), the magnetic tip may be retracted or withdrawn a distance (e.g., to a location prior to the internal junction), the external magnetic member may be repositioned relative to the patient, and the magnetic tip may be again translated beyond the internal junction of the patient and into the second passageway. The echogenic member or echogenic portion may be visualized again to confirm the location of the magnetic tip within the second passageway prior to further advancement.

## Claims

1. An apparatus, comprising:
an elongated tube (210) having a first end (211), a second end (213), and defining a first lumen (212) and a second lumen, the first lumen (212) extending from the first end to the second end;
a magnetic tip coupled to the first end (211) of the elongated tube (210), the magnetic tip being tapered toward a distal end of the magnetic tip; and
an inflatable member disposed within the elongated tube (210) proximal of the tapered magnetic tip and fluidically coupled to the second lumen such that the inflatable member can receive fluid via the second lumen.

2. The apparatus of claim 1, wherein the magnetic tip is configured and oriented such that the magnetic tip can apply a force in a first direction lateral to a central axis of the elongated tube such that the magnetic tip can be moved in a second direction opposite the first direction by an oppositely polarized magnetic member disposed a distance from the magnetic tip along the first direction.

3. The apparatus of claim 1, wherein the elongated tube has a sidewall and defines at least one aperture in the sidewall such that the first lumen is in fluid communication with the at least one aperture.

4. The apparatus of claim 1, wherein the elongated tube defines at least one opening such that fluid can be delivered through the elongated tube and out of the at least one opening.

5. The apparatus of claim 1, wherein the magnetic tip includes oppositely-disposed sidewalls and a bottom surface, the bottom surface extending parallel to the central axis of the elongated tube, the oppositely-disposed sidewalls tapering toward the bottom surface.

6. A system, comprising:
a magnetically responsive assembly, including the apparatus of claim 1; and
a magnetic member configured to be disposed adjacent a surface of a patient and to apply a magnetic field such that, as the magnetic tip of the magnetically responsive assembly is translated through a first passageway toward an internal junction of the patient in which the first passageway, a second passageway, and a third passageway intersect, a magnetic force produced by the interaction of the magnetic field of the magnetic member and a magnetic field of the magnetic tip can urge the position of the magnetic tip to align the magnetic tip with the second passageway.

7. The system of claim 6, wherein the magnetic member and the magnetic tip can be arranged in the same plane as a central axis of the first passageway, a central axis of the second passageway, and a central axis of the third passageway.

8. The system of claim 6, wherein the magnetic member and the magnetic tip can be arranged such that a line passing through both the magnetic member and the magnetic tip can intersect a central axis of the second passageway and the third passageway when the magnetic tip is positioned in the second passageway.

9. The system of claim 6, wherein the first passageway is a first portion of a hypopharynx of a patient, the second passageway is a second portion of the hypopharynx, the second portion of the hypopharynx coupled to an esophagus of the patient, and the third portion is a larynx of the patient, and the magnetic member is configured to maintain the magnetic tip into contact with a posterior hypopharynx wall of the patient as the magnetic tip translates through the hypopharynx and into the esophagus of the patient.

10. The apparatus of claim 1, wherein the magnetic tip includes an opening such that the first lumen can be in fluid communication with the stomach via the opening when the first end of the elongated tube is disposed within the stomach.

11. The apparatus of claim 5, wherein the distal end of the magnetic tip includes a straight edge disposed perpendicular to a central axis of the first end of the elongated tube, each of the oppositely-disposed sidewalls including a parallel-extending portion that extends parallel to the central axis and a diagonal-extending portion that extends toward the central axis from the parallel-extending portion to the straight edge and tapers toward the straight edge.

12. The apparatus of claim 1, wherein the magnetic tip is spatula-shaped.

13. The apparatus of claim 1, wherein the magnetic tip has a first side and a second side, the first side having an opposite polarity from the second side.

## Patentansprüche

1. Vorrichtung, die Folgendes umfasst:
eine langgestreckte Röhre (210), die ein erste Ende (211) und ein zweites Ende (213) aufweist und ein erstes Lumen (212) und ein zweites Lumen definiert, wobei sich das erste Lumen (212) von dem ersten Ende zu dem zweiten Ende erstreckt;
eine magnetische Spitze, die an das erste Ende (211) der langgestreckten Röhre (210) gekoppelt ist, wobei die magnetische Spitze in Richtung eines distalen Endes der magnetischen Spitze konisch zuläuft; und
ein aufblasbares Element, das innerhalb der langgestreckten Röhre (210) in der Nähe der konisch zulaufenden magnetischen Spitze angeordnet ist und an das zweite Lumen derart fluidtechnisch gekoppelt ist, dass das aufblasbare Element ein Fluid über das zweite Lumen empfangen kann.

2. Vorrichtung nach Anspruch 1, wobei die magnetische Spitze derart konfiguriert und orientiert ist, dass die magnetische Spitze eine Kraft in einer ersten Richtung lateral zu einer mittleren Achse der langgestreckten Röhre derart ausüben kann, dass die magnetische Spitze in einer zweiten Richtung entgegengesetzt zu der ersten Richtung durch ein entgegengesetzt polarisiertes magnetisches Element, das in einem Abstand von der magnetischen Spitze entlang der ersten Richtung angeordnet ist, bewegt werden kann.

3. Vorrichtung nach Anspruch 1, wobei die langgestreckte Röhre eine Seitenwand besitzt und mindestens eine Öffnung in der Seitenwand definiert, so dass das erste Lumen mit der mindestens einen Öffnung in Fluidkommunikation ist.

4. Vorrichtung nach Anspruch 1, wobei die langgestreckte Röhre mindestens eine Öffnung definiert, so dass Fluid durch die langgestreckte Röhre und aus der mindestens einen Öffnung heraus geliefert werden kann.

5. Vorrichtung nach Anspruch 1, wobei die magnetische Spitze gegenüberliegend angeordnete Seitenwände und eine untere Fläche enthält, wobei sich die untere Fläche parallel zu der mittleren Achse der langgestreckten Röhre erstreckt, wobei die gegenüberliegend angeordneten Seitenwände in Richtung der unteren Fläche zulaufen.

6. System, das Folgendes umfasst:
eine magnetisch reagierende Anordnung, die die Vorrichtung nach Anspruch 1 enthält; und
ein magnetisches Element, das konfiguriert ist, angrenzend an eine Fläche eines Patienten angeordnet zu werden und ein magnetisches Feld derart anzulegen, dass dann, wenn die magnetisch reagierende Anordnung durch einen ersten Durchgang in Richtung einer inneren Verzweigungsstelle des Patienten, in der sich der erste Durchgang, ein zweiter Durchgang und ein dritter Durchgang schneiden, verschoben wird, eine magnetische Kraft, die durch die Wechselwirkung des Magnetfeldes des magnetischen Elements und eines Magnetfeldes der magnetischen Spitze erzeugt wird, die Position der magnetischen Spitze drängen kann, die magnetische Spitze auf den zweiten Durchgang auszurichten.

7. System nach Anspruch 6, wobei das magnetische Element und die magnetische Spitze in derselben Ebene wie eine mittlere Achse des ersten Durchgangs, eine mittlere Achse des zweiten Durchgangs und eine mittlere Achse des dritten Durchgangs angeordnet werden können.

8. System nach Anspruch 6, wobei das magnetische Element und die magnetische Spitze derart angeordnet werden können, dass eine Linie, die sowohl durch das magnetische Element als auch die magnetische Spitze führt, eine mittlere Achse des zweiten Durchgangs und des dritten Durchgangs schneiden kann, wenn die magnetische Spitze in dem zweiten Durchgang positioniert wird.

9. System nach Anspruch 6, wobei der erste Durchgang ein erster Teil eines Hypopharynx eines Patienten ist, der zweiten Durchgang ein zweiter Teil des Hypopharynx ist, der zweite Teil des Hypopharynx an einen Ösophagus des Patienten gekoppelt ist und der dritte Teil ein Larynx des Patienten ist und das magnetische Element konfiguriert ist, die magnetische Spitze in Kontakt mit einer hinteren Hypopharynx-Wand des Patienten zu halten, während die magnetische Spitze durch den Hypopharynx und in den Ösophagus des Patienten verschoben wird.

10. Vorrichtung nach Anspruch 1, wobei die magnetische Spitze eine Öffnung aufweist, so dass das erste Lumen mit dem Magen über die Öffnung in Fluidkommunikation sein kann, wenn das erste Ende der langgestreckten Röhre innerhalb des Magens angeordnet ist.

11. Vorrichtung nach Anspruch 5, wobei das distale Ende der magnetischen Spitze einen geraden Rand aufweist, der senkrecht zu einer mittleren Achse des ersten Endes der langgestreckten Röhre angeordnet ist, wobei jede der entgegengesetzt angeordneten Seitenwände einen sich parallel erstreckenden Teil, der sich parallel zu der mittleren Achse erstreckt, und einen sich diagonal erstreckenden Teil, der sich in Richtung der mittleren Achse von dem sich parallel erstreckenden Teil zu dem geraden Rand erstreckt und in Richtung der geraden Kante zuläuft, enthält.

12. Vorrichtung nach Anspruch 1, wobei die magnetische Spitze spachtelförmig ist.

13. Vorrichtung nach Anspruch 1, wobei die magnetische Spitze eine erste Seite und eine zweite Seite besitzt, wobei die erste Seite eine zu der zweiten Seite entgegengesetzte Polarität aufweist.

## Revendications

1. Appareil, comprenant :
un tube allongé (210) doté d'une première extrémité (211), d'une deuxième extrémité (213), et définissant une première lumière (212) et une deuxième lumière, la première lumière (212) s'étendant depuis la première extrémité jusqu'à la deuxième extrémité ;
un embout magnétique accouplé à la première extrémité (211) du tube allongé (210), l'embout magnétique s'effilant vers une extrémité distale de l'embout magnétique ; et
un élément gonflable agencé à l'intérieur du tube allongé (210) à proximité de l'embout magnétique effilé et accouplé fluidiquement à la deuxième lumière de façon à permettre à l'élément gonflable de recevoir du fluide via la deuxième lumière.

2. Appareil selon la revendication 1, l'embout magnétique étant configuré et orienté de façon à permettre à l'embout magnétique d'appliquer une force dans une première direction latérale par rapport à un axe central du tube allongé de façon à permettre à l'embout magnétique de se déplacer dans une deuxième direction opposée à la première direction sous l'action d'un élément magnétique de polarisation opposée agencé à distance de l'embout magnétique suivant la première direction.

3. Appareil selon la revendication 1, le tube allongé étant doté d'une paroi latérale et définissant au moins une ouverture dans la paroi latérale de façon à mettre la première lumière en communication fluidique avec l'au moins une ouverture.

4. Appareil selon la revendication 1, le tube allongé définissant au moins une ouverture de façon à permettre l'acheminement de fluide à travers le tube allongé et sa sortie par l'au moins une ouverture.

5. Appareil selon la revendication 1, l'embout magnétique comportant des parois latérales agencées à l'opposé l'une de l'autre et une surface inférieure, la surface inférieure s'étendant parallèlement à l'axe central du tube allongé, les parois latérales à l'opposé l'une de l'autre s'effilant vers la surface inférieure.

6. Système, comprenant :
un ensemble magnétiquement réactif, comportant l'appareil selon la revendication 1 ; et
un élément magnétique configuré pour être agencé adjacent à une surface d'un patient et pour appliquer un champ magnétique pour faire en sorte que, tandis que l'embout magnétique de l'ensemble magnétiquement réactif se déplace en translation à travers un premier passage vers une jonction interne du patient où se coupent le premier passage, un deuxième passage et un troisième passage, une force magnétique produite par l'interaction du champ magnétique de l'élément magnétique et d'un champ magnétique de l'embout magnétique amène l'embout magnétique à se positionner pour aligner l'embout magnétique avec le deuxième passage.

7. Système selon la revendication 6, l'élément magnétique et l'embout magnétique étant susceptibles d'être agencés dans le même plan qu'un axe central du premier passage, un axe central du deuxième passage, et un axe central du troisième passage.

8. Système selon la revendication 6, l'élément magnétique et l'embout magnétique étant susceptibles d'être agencés pour faire en sorte qu'une ligne passant à la fois par l'élément magnétique et par l'embout magnétique coupe un axe central du deuxième passage et du troisième passage lorsque l'embout magnétique est positionné dans le deuxième passage.

9. Système selon la revendication 6, le premier passage constituant une première partie de l'hypopharynx d'un patient, le deuxième passage constituant une deuxième partie de l'hypopharynx, la deuxième partie de l'hypopharynx étant reliée à l'œsophage du patient, et la troisième partie constituant le larynx du patient, et l'élément magnétique étant configuré pour maintenir l'embout magnétique au contact de la paroi postérieure de l'hypopharynx du patient tandis que l'embout magnétique se déplace en translation à travers l'hypopharynx et jusque dans l'œsophage du patient.

10. Appareil selon la revendication 1, l'embout magnétique comportant une ouverture pour faire en sorte de mettre la première lumière en communication fluidique avec l'estomac via l'ouverture lorsque la première extrémité du tube allongé est agencée à l'intérieur de l'estomac.

11. Appareil selon la revendication 5, l'extrémité distale de l'embout magnétique comportant un bord droit agencé perpendiculairement à un axe central de la première extrémité du tube allongé, chacune des parois latérales agencées à l'opposé l'une de l'autre comportant une partie s'étendant parallèlement qui s'étend parallèlement à l'axe central et une partie s'étendant diagonalement qui s'étend vers l'axe central depuis la partie s'étendant parallèlement jusqu'au bord droit et s'effile vers le bord droit.

12. Appareil selon la revendication 1, l'embout magnétique prenant la forme d'une spatule.

13. Appareil selon la revendication 1, l'embout magnétique étant doté d'un premier côté et d'un deuxième côté, le premier côté étant doté d'une polarité opposée à celle du deuxième côté.
